# EUROPEAN PATENT APPLICATION

(11) **EP 3 403 730 A1**
(43) Date of publication of application: **21.11.2018**
(21) Application number: 17171540.2
(22) Date of filing: 17.05.2017
(51) Int. Cl.: B05C 17/005, A61M 5/28, A61J 7/00

(54) **DISCHARGER AND METHOD OF DISCHARGING**

(71) Applicant: Sulzer Mixpac AG, 9469 Haag (Rheintal) (CH)
(72) Inventor: Kreutzmann, Vera, 8865 Bilten (CH); Hässig, Andreas, 8739 Rieden (CH); Eggmann, Marc, 8865 Bilten (CH); Sokullu, Toprak, 9100 Herisau (CH)
(74) Representative: Manitz Finsterwald Patentanwälte PartmbB

(57) **Abstract**

The present invention relates to a discharger that comprises a dispensing element and a housing, the housing having a static piston connected to the dispensing element at least in a fluid conducting manner and with the static piston having a piercing tip and being arranged within the housing. The invention further relates to a method of discharging a fluid from a discharger.

## Description

The present invention relates to a discharger that comprises a dispensing element and a housing, the housing having a static piston connected to the dispensing element at least in a fluid conducting manner and with the static piston having a piercing tip and being arranged within the housing. The invention further relates to a method of discharging a fluid from a discharger.

Dischargers are used to dispense pre-defined amounts of fluids from a container connectable or connected to the discharger. In this connection the container is typically sealed off at one end thereof by a seal prior to use. On use of the discharger the container is moved towards the housing and the seal of the container is pierced in order to permit the fluid stored in the container to be discharged out of the container and from the discharger via a dispensing element.

During the piercing action, a static piston arranged within the housing initially engages the seal of the container resulting in a pressure on the seal of the container leading to an increase in pressure of the fluid stored therein, prior to the actual piercing of the seal. Once pierced, this then pressurized fluid, particularly if it is a low viscous fluid, such as a tick or flea drug for animals, for example a tick or flea drug sold by the company Merial using the trademark frontline, can leak out from the container via the discharger, if the container is not mounted correctly at the housing. These leaks can contaminate areas not intended to come into contact with the fluid.

In view of this background it is an object of the invention to avoid fluid from leaking from a discharger in an uncontrolled manner following a piercing of its seal. It is a further object of the present invention to ensure a correct installation of the container at the discharger. It is yet a further object of the invention to minimize a residue of fluid remaining in the discharger once the discharging has taken place.

These objects are satisfied by a discharger having the features of claim 1.

Such a discharger comprises a dispensing element and a housing, the housing having a static piston connected to the dispensing element at least in a fluid conducting manner and with the static piston having a piercing tip and being arranged within the housing; the discharger being configured to receive at least a portion of a container for containing a fluid, with the container then being moveable relative to the static piston and the housing along a longitudinal direction of the discharger, wherein the housing comprises a sealing element arranged at an inner surface of the housing, with the sealing element projecting from the inner surface of the housing in the direction of the longitudinal direction of the discharger.

In use of the discharger, the sealing element arranged at the inner surface of the housing is configured to engage a surface of a container in particular when the discharger is in one of the discharging state and the discharged state. This sealing element is provided to prevent the fluid from exiting between the housing and the container. This is the region of the discharger where a user has his/her hands during discharging. This means that, at least during discharging, a user does not come into direct contact with the fluid present in the discharger, as leaks in this region can be prevented by the sealing element present at the inner surface of the housing.

The present invention thus directly seals between the parts of the discharger moveable relative to one another where the presence of a leak is most likely. This is achieved in a cost effective manner through the provision of a seal at the inner surface of the housing.

Preferably the housing has a cylindrical wall having the inner surface and the sealing element is arranged to circumferentially extend around the inner surface of the cylindrical wall. A cylindrical housing is simple to manufacture e.g. in injection molded processes. Providing a sealing element that extends around the complete inner surface of such a housing means that an effective seal is formed without interruptions.

Moreover, round parts can be aligned accurately relative to one another permitting a simple mounting of the container at the housing of the discharger. Such a design of housing can thus advantageously also facilitate the mounting of the container at the discharger.

It is preferred if the sealing element is arranged at an inner surface of the housing in the region of the piercing tip. On use of the discharger the forces that arise during the piercing action of the seal cause the fluid stored in the container to leak out in the region of the piercing tip. By providing the sealing element in the region of the piercing element thus means that it is placed in the vicinity of the part of the housing where a leak is most likely to arise.

Particularly preferably the sealing element is an inwardly projecting sealing lip. A sealing lip is simple and cost effective to manufacture and ensures a good seal between two components moveable relative to one another.

Advantageously the sealing element projects in the direction of the piercing tip arranged along the longitudinal direction. In this way the sealing element is provided in direct vicinity of the part of the housing where a leak is most likely to arise.

Preferably the sealing element is arranged in a plane perpendicular to the longitudinal direction, with the plane further comprising at least one of the piercing tip, an end of the piercing tip and an inlet of the piercing tip. Such a design has found to yield particularly good sealing results.

It is preferred if the sealing element is either integrally formed at the inner surface of the cylindrical wall or is in contact with the inner surface of the cylindrical wall. An integrally formed sealing element is simple to manufacture e.g. during an injection molding process and ensures an intimate contact of the sealing element at the housing. A separate sealing element, such as an O-ring, can likewise be employed in a simple and cost effective manner.

Advantageously the discharger further comprises the container, wherein the sealing element is only in contact with or connected to the housing in a storage state of the discharger; and wherein the sealing element is configured to engage the container in at least one of a discharging state and a discharged state of the discharger in addition to being in contact with or connected to the housing in both the discharging state and the discharged state of the discharger. In this way a discharger is formed in which forces only act on the sealing element when the discharger has been displacement from the storage state into one of the discharging and discharged state of the discharger. Such forces could lead to a deterioration over time of the at least one sealing element and hence to a reduced sealing action in this region on use of the discharger when the discharger is in one of the discharging state and the discharged state.

Preferably the container comprises a seal, with the piercing tip being configured to pierce the seal of the container when the container is moved out of the storage state and towards the housing, and in particular wherein the sealing element is configured to engage the container, preferably a surface of the container, as soon as the seal of the container is in contact with the piercing tip.

Using a seal the container can be used to store fluids. By permitting the seal to only be opened once the discharger is activated, the fluid stored therein will not be subjected to the surrounding atmosphere prior to activation of the discharger. Moreover, by positioning the sealing element within the housing such that it only comes into contact with the container once the seal is pierced, no forces act on the sealing element prior to activation of the discharger ensuring that the sealing element does not deteriorate during storage of the discharger.

Advantageously the static piston comprises at least one further sealing element arranged at the piercing tip. Such additional sealing elements further improve the leak tightness of the discharger.

Preferably the at least one further sealing element provides a seal between the piercing tip and an inner surface of the container when the moveable container is moved relative to the housing into a position in which the seal is pierced, with said inner surface being different from the surface of the container

The at least one further sealing element of the static piston thereby provides a direct seal between the static piston and the container ensuring that no fluid can pass between the at least one sealing element and an inner surface of the container and thereby preferably prevent the fluid from leaking from the container into a part of the housing that is not configured to receive a fluid during either the discharging or discharged state.

In this connection it should be noted that the at least one further sealing element could also be a sealing lip. A sealing lip is simple and cost effective to manufacture and ensures a good seal between two components moveable relative to one another.

It is preferred if the container comprises a compartment in its interior in which the fluid is initially stored prior to piercing the seal, and wherein said surface that is engaged by the sealing element of the housing is an outer surface of a wall of the compartment.

Advantageously the container further comprises an outer wall arranged in parallel to the wall of the compartment and a guide groove present between the outer wall and the wall of the compartment, with said guide groove being configured to receive at least some of the housing.

The guide groove is provided at such a double walled container as a mounting guide. Thereby a skew mounting of the container at a housing of a discharger can be avoided. Moreover, in use of the discharger the groove acts as a guide groove. This is because the housing can be guided in the groove relative to the container, when the container is mounted at a discharger.

Preferably the discharger comprises a child safety lock. Such child safety locks prevent the accidental activation of the discharger.

It is preferred if a fluid is stored in the container in the storage state of the discharger. In this case substances such as frontline can be readily stored in the container. In this connection it should be noted that the container is advantageously sealed off by the seal that is preferably configured as a membrane. A membrane is an advantageous kind of seal that following its piercing enables the correct interaction of the container with the discharger.

Advantageously the discharger is filled with a fluid selected from the group of members consisting of a topical medication, a medical fluid, a cosmetic and/or skin care preparation, a dental fluid, a veterinary fluid, an adhesive fluid, a disinfectant fluid, and combinations of the foregoing.

In this way, for example, bleaching agents for teeth, fluoride for teeth, disinfectants, adhesives, wound adhesives, topical anesthetics, sunscreen, after sun, skin moisturizers or other drugs and cosmetics can be stored in the discharger and administered using the discharger. Such fluids can e.g. be the aforementioned frontline, eye drops and nose drops used to e.g. decongest eyes and noses of patients suffering from allergies, colds or flues.

In a further aspect the present invention relates to a method of discharging a fluid from a discharger, in particular a discharger as discussed in the foregoing, the method comprising the steps of:
- guiding a container in a longitudinal direction of the discharger towards a piercing tip of a static piston accommodated within a housing of the discharger,
- piercing a seal present at the container by means of the piercing tip causing the seal to be pierced, in particular initially at a center of the seal,
- bringing an outer surface of the container into engagement with at least one sealing element arranged at an inner surface of the housing to prevent the fluid from passing between the at least one sealing element and the outer surface, and preferably
- guiding the container further towards the static piston to discharge the fluid via an outlet of the discharger.

The advantages associated with the discharger in accordance with the invention likewise hold true for the method described herein.

Further embodiments of the invention are described in the following description of the Figures. The invention will be explained in the following in detail by means of embodiments and with reference to the drawing in which is shown:
- Fig. 1: a perspective view of a discharger in a storage state;
- Fig. 2: a perspective view of the discharger of Fig. 1 in a discharged state;
- Fig. 3: a part sectional view of the discharger in the state of Fig. 1;
- Fig. 4: a part sectional view of the discharger in the state of Fig. 2; and
- Fig. 5: a sectional view of a further type of static piston of the discharger.

In the following the same reference numerals will be used for parts having the same or equivalent function. Any statements made having regard to the direction of a component are made relative to the position shown in the drawing and can naturally vary in the actual position of application.

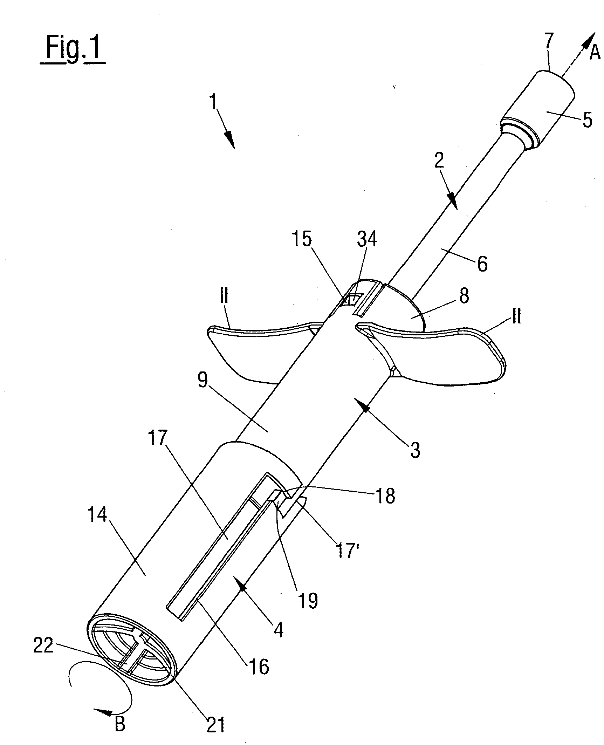

Fig. 1 shows a discharger 1 in a storage state. The discharger 1 comprises a dispensing element 2, a housing 3 and a container 4. The dispensing element 2 extends between a distal end 5 and a proximal end 6 and thereby defines a longitudinal direction A respectively a longitudinal axis A of the discharger 1, with an outlet 7 of the discharger 1 being configured at the distal end 5. The outlet 7 can either be a simple nozzle (not shown) or comprise a specifically designed nozzle (also not shown) that enables specific spraying actions from being undertaken with the discharger 1.

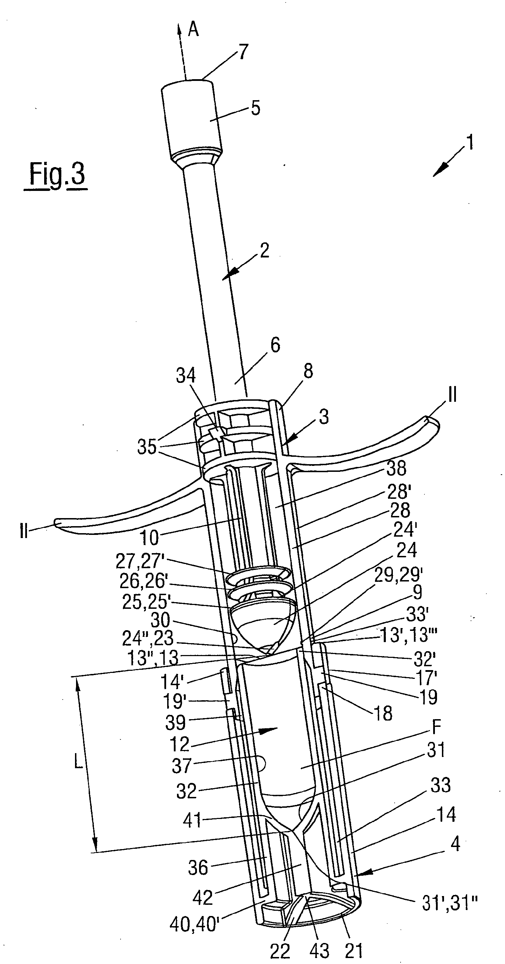

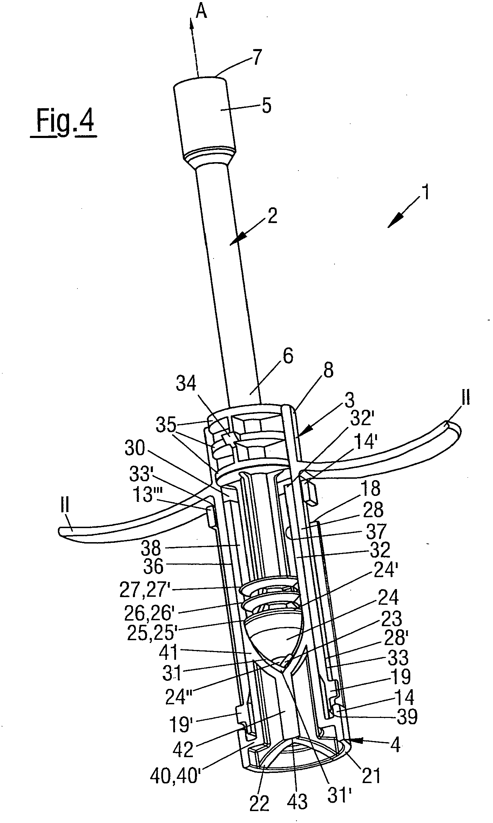

The housing 3 has a distal region 8 and a proximal region 9, with the distal region 8 of the housing 3 adjoining the proximal end 6 of the dispensing element 2. The housing 3 comprises a static piston 10 (see e.g. Figs. 3 and 4) arranged within it and has wing-like projections 11 projecting away from the generally cylindrical shaped housing 3 in the distal region 8 of the housing 3.

The housing 3 further comprises a cut-out 15 in the distal region 8. Moreover, a nose 34 can be seen that cooperates with this cut-out. The cut-out 15 and nose 34 form parts of a snap-in connection as will be explained in the following.

The container 4 is arranged at the proximal region 9 of the housing 3. The container 4 comprises a compartment 12 in which a fluid F is stored and sealed off with respect to the static piston 10 by means of a seal 13" that is configured as a membrane 13 (see Fig. 3).

In the present example the outer wall 14 of the container 4 comprises a slot 16 having two longitudinal sections 17, 17' offset in parallel to one another and to the longitudinal direction A and connected to one another via a connection section 18. In the storage state of the discharger 1 shown in Fig. 1, a pin 19 projecting from the proximal region 9 of the housing 3 is present in the connection section 18.

In the present example, the pin 19 is placed in the connection section 18 of the slot 16. By placing the pin 19 in the connection section 18 that does not extend in the direction of the longitudinal axis A an accidental linear displacement of the container 4 relative to the housing 3 and thereby either an accidental removal of the container 4 from the housing 3 or an accidental movement of the container 4 towards the wing-like projections 11 can be prevented. In this way the discharger 1 comprises a child safety lock, with the child safety lock being formed between the container 4 and the housing 3.

On a desired activation of the discharger 1 the container 4 is rotated in the direction of the arrow B, so that the pin 19 is then guided into the longitudinal section 17. Once the pin 19 is present in the longitudinal section 17, the container 4 can be pressed towards the wing-like projections 11 in the direction of the longitudinal axis A. A user can carry out the pressing of the container 4 towards the wing-like projections 11 by placing a thumb or a different finger at an end 21 of the housing and two further fingers at each of the wing-like projections 11 and then move these fingers and/or the thumb towards one another in a clamping like manner. The end 21 has a recess 22 formed therein for an improved placement of the finger/thumb at the end 21 of the container 4.

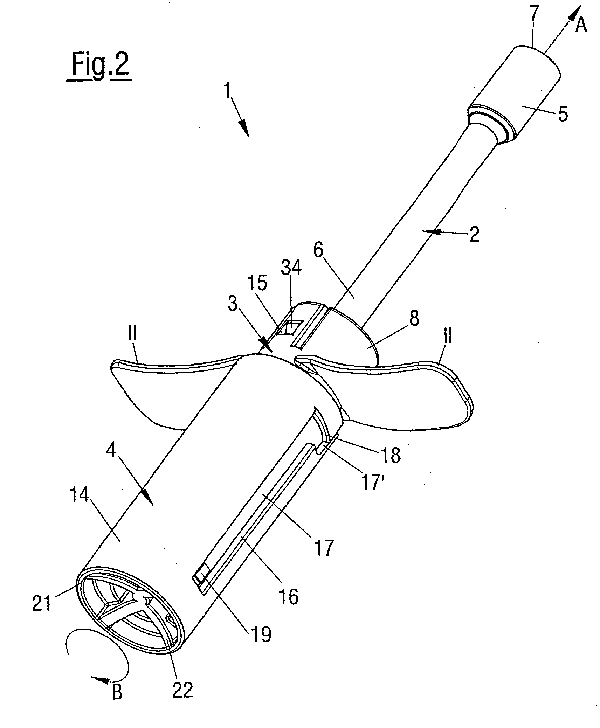

Fig. 2 shows the discharger of Fig. 1 in the discharged state, i.e. the state in which the user has pressed the container 4 from the proximal region 9 of the housing 3 up to the wing-like projections 11 formed at the housing 3. In the discharged state of the discharger 1, the fluid F stored in the compartment 12 has been discharged from the discharger 1 via the outlet 7 (see Fig. 4).

Fig. 3 shows a part sectional view of the discharger 1 shown in the state shown in Fig. 1. This view shows how the static piston 10 is arranged within the housing 3 of the discharger 1. The static piston 10 is connected to the dispensing element 2 in a fluid conducting manner.

For this purpose the static piston 10 has an inlet 23 that leads into a passage (not shown) that connects the inlet 23 with the outlet 7 of the dispensing element 2. The passage thereby extends through the static piston 10 and the dispensing element 2.

The discharger 1 is preferably designed such that the only way a fluid present in the container 4 can pass to the outside via the distal region 8 is via the inlet 23 and the passage, otherwise the housing 3 comprises sealing elements 25', 26', 27', 29' to seal off the housing 3 towards the outside.

The static piston 10 comprises a piercing tip 24 having the inlet 23 formed at one end 24" of the piercing tip 24. The other end 24' of the piercing tip 24 comprises a first sealing lip 25 as a first sealing element 25'. A second sealing lip 26 and a third sealing lip 27 are likewise formed at the static piston 10, as second and third sealing elements 26', 27'. The first, second and third sealing lips 25, 26, 27 are arranged one after the other in parallel to one another in the direction of the longitudinal axis A of the discharger 1. In the storage state of the discharger 1 shown in Fig. 3 the first, second and third sealing lips 25, 26, 27 do not contact a cylindrical wall 28 of the housing 3.

The cylindrical wall 28 of the housing 3 comprises the sealing element 29'. In this instance the sealing element 29' is a sealing lip 29 that circumferentially extends around an inner surface 30 of the cylindrical wall 28 in the proximal region 9 of the housing 3. In the storage state shown in Fig. 3 the sealing lip 29 is an inwardly projecting sealing lip 29. The sealing lip 29 projects, on the one hand, in the direction of the piercing tip 24 arranged around the longitudinal axis A and, on the other hand, into the interior of the housing 3 without engaging a further component. This means that the sealing lip 29 faces the longitudinal axis A of the discharger 1, preferably such that it does not contact nor connect with any further components other than the housing 3 in the storage state of the discharger 1.

It should be noted that a plane comprising the piercing tip 24, the inlet 23 and the end 24" also comprises the sealing element 29'. This plane is arranged perpendicular to the longitudinal direction A.

Fig. 3 also shows a section through the container 4 of the discharger 1 in a plane perpendicular to the longitudinal direction A. As mentioned in connection with Fig. 1, the container 4 comprises the compartment 12 for containing the fluid F in the storage state. The compartment 12 extends from a seal formed by the membrane 13 to a piercing tip receiving end 31, also referred to as an end 31' of the compartment 12, in the direction towards the end 21 of the container 4 comprising the recess 22.

The end 31' of the compartment 12 that is oppositely disposed of the membrane 13 is formed by a wall 41 of the compartment 12. The wall 41 converges from the wall 32 to a common point 31" at the end 31' coinciding with the longitudinal axis A to form the end 31' of the compartment 12. This means that a diameter of the compartment 12 reduces from the wall 32 to the longitudinal axis A along the wall 41.

As also shown the end 31' of the compartment 12 is set back from the rear end 21 of the container 4 by approximately 30% of a length of the outer wall 14. In this connection it should be noted that the end 31' can be set back from the rear end 21 by at least 20%, in particular by 25 to 45%, of the length of the outer wall 14.

A further web 42 is provided in the region of the rear end 21 of the container. This further web 42 extends in parallel to the outer wall 14 between the end 31' of the compartment 12 and the rear end 21 of the container 4. This further web 42 forms a base 43 of the recess 22 at the rear end 21 and hence a base 43 where a finger or thumb can beneficially be placed.

A size of the compartment 12 defines the volume of fluid F that can be stored in the container 4. This means that if a lesser volume of fluid F is to be stored within the compartment 12, then the length L of the compartment 12 can be selected shorter. Consequently, if a greater volume of fluid F is to be stored in the container 4 then a length L of the compartment 12 can be selected longer than in the present case. Hence the volume of fluid F stored in the container 4 corresponds to a space of the container 4.

In this connection it should be noted that typical filling volumes of the compartment 12 of the container 4 are 0.1 to 10 ml, preferably 0.2 to 5 ml.

It should further be noted that a thickness of the wall 32 of the compartment 12 is typically selected in the range of 0.7 to 1.5 mm, preferably in the range of 0.9 to 1.1 mm and especially of around 1 mm. Likewise a thickness of the outer wall 14 of the container 4 is typically selected in the range of 0.7 to 1.5 mm, preferably in the range of 0.9 to 1.1 mm and especially of around 1 mm.

In this connection it should be noted that a thickness of the wall 28 of the housing 3 is typically selected in the range of 0.7 to 1.5 mm, preferably in the range of 0.9 to 1.1 mm and especially of around 1 mm.

It should be noted in this connection that if a compartment 12 of greater volume is selected then a length of the static piston 10 can also be increased in order to ensure that as much as possible of the fluid F initially stored in the compartment 12 is discharged from the discharger 1.

In the embodiment previously described the compartment 12 is integrally formed with the container 4, i.e. the container is a single-part container 4. In a further embodiment a separate compartment in the form of carriage (not shown) having a pre-defined volume could be used to form a two-part or multi-part container (also not shown). The sealing elements 25', 26', 27' are then configured to interact with an inner surface of this multi-part container, i.e. with an inner surface of the carriage.

It should further be noted that the length L of the compartment 12 is defined as the distance between the membrane 13 and the piercing tip receiving end 31.

The shape of the piercing tip receiving end 31 is selected to be complementary to the shape of the piercing tip 24. This is because on discharging the fluid F stored in the compartment 12 of the container 4 as little residue of the fluid F as possible is desired, preferably such that all of the fluid F stored in the container 4 is discharged once the discharger is in the discharged state shown in Fig. 4.

As also shown in Fig. 3, the container 4 is a double walled container comprising an inner wall 32 and the outer wall 14 separated by a, preferably annular, guide groove 33. The inner wall 32 forms a wall of the compartment 12. The guide groove 33 can also be referred to as a ring-like gap that is formed between the inner wall 32 and the outer wall 14 of the double walled container. It should be noted in this connection that the at least one slot 16 extends from the guide groove 33 to an outer surface 14" of the outer wall 14. Preferably the inner wall 32 and the outer wall 14 are injection molded in one piece during the manufacture of the container 4, with a connecting web 40 also being formed during the injection molding process connecting the inner wall 32 to the outer wall 14 at an end 40' of the guide groove 33.

In this connection it should be noted that a length or depth of the guide groove 33 in the longitudinal direction A of the discharger 1 is approximately 90% of a length of the outer wall 14 of the discharger 1 of Fig. 3. It should further be noted that the length of the guide groove 33 can be selected to correspond to 50% to 95% of a length of the outer wall 14.

In the present example the membrane 13 also forms a front end 13'" of the compartment 12 and thereby of the inner wall 32. The membrane 13 not only forms the front end of the compartment 12, but also defines the front end of the double barreled container 4, as the plane 13' comprising the membrane 13 defines said front end. The plane 13' thereby in addition to the membrane 13 also comprises an end 14' of the outer wall 14 and an end 32' of the wall 32. Thus, the double barreled container 4 extends from the membrane 13 to the end 21, i.e. the rear end 21, having the recess 22 formed therein.

It should be noted in this connection that an opening 33' of the groove is arranged at the front end 13'" of the container, i.e. the groove 33 is open towards the housing 3 in order to receive the cylindrical wall 28 at the front end 13'" of the container.

In the storage state of the discharger 1 shown in Fig. 3, the front end of the compartment 12 is received within the housing 3, whereas the outer wall 14 of the double barreled container 4 surrounds the cylindrical wall 28 of the housing 3 in the proximal region 9 thereof. This means that the front end of the container 4 is configured to receive at least the proximal region 9 of the housing 3.

The section through the container 4 of Fig. 3 also shows the presence of a second pin 19' at the other side of the housing 3. This second pin 19' also cooperates with a second slot (not shown) similar to slot 16 present in the double walled container 4.

In the present case the first pin 19 and the second pin 19' are arranged at 180° with respect to one another at an outer surface 28' of the housing 3. In practice dischargers are possible that utilize only one pin and slot arrangement or also more than two pin and slot arrangements. It is also conceivable that the pins are not arranged at 180° with respect to one another at the outer surface 28' of the housing 3, but at a different angle to thereby ensure a correct alignment of the container 4 relative to the housing 3. As can further be seen from Fig. 3 the pins 19, 19' are set back from an end 39 of the housing 3 in the proximal region 9.

On assembly of the discharger 1 the different components are connected to one another. It should be noted in this connection that the static piston 10 and the dispensing element 2 are preferably injection molded in one piece in a common mold in a preferred design of the discharger 1. Alternatively, an overmolding process could also be employed.

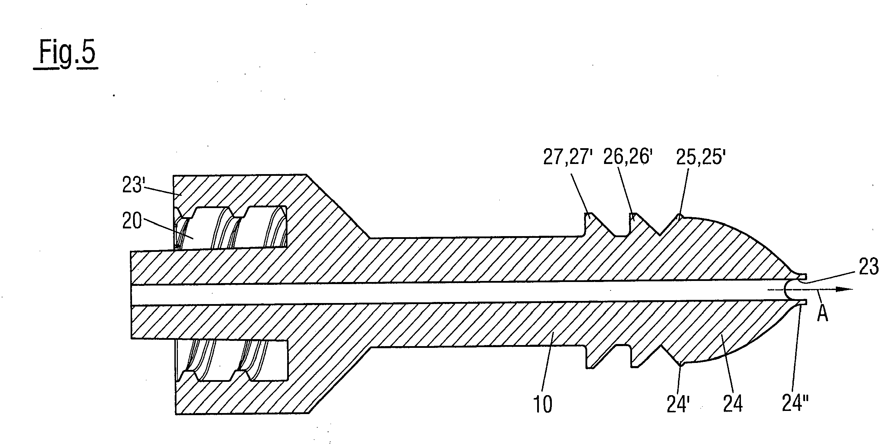

Alternatively the individual components of the discharger 1, e.g. the dispensing element 2, the housing 3, the container 4, the static piston 10, can also be formed separately and then assembled, for example, the static piston 10 and the dispensing element 2 can be connected to one another via a Luer lock connection (see e.g. Fig. 5 in this connection).

In this connection it should be noted that the components of the discharger can be formed from polymeric materials, such as PE (polyethylene), PP (polypropylene) and COC (cyclic olefin copolymers).

For example, the container 4 can be formed by a polymeric material such as COC.

In this connection it should also be noted that like the assembly comprising the static piston 10 and the dispensing element 2, the housing 3 and the container 4 can also be formed in specifically designed molds (not shown) in an injection molding process as separate parts.

The assembly comprising the static piston 10 and the dispensing element 2 is then inserted into the housing 3 via the distal region of the housing 3. The assembly comprising the static piston 10 and the dispensing element 2 is fixed to the inner surface 30 of the housing 3, on the one hand, via snap-in connections, in this respect the snap-in connection is formed by the nose 34 that engages the cut-out 15 (see Fig. 1) present in the cut away section of the housing 3. A second snap-in connection is also present at the other side of the housing 3 that cannot be seen in the depicted section.

On the other hand, the assembly comprising the static piston 10 and the dispensing element 2 is fixed to the inner surface 30 of the housing 3 by means of a press fit. For this purpose three rings 35 are provided at the assembly comprising the static piston 10 and the dispensing element 2. In addition to ensuring a press fit, these three rings 35 also ensure the correct parallel orientation of the static piston 10 within the housing 3, i.e. that the static piston 10 is not inserted at a skew angle into the housing 3.

It should be noted in this connection, that the three rings 35, that provide the correct orientation of the static piston 10 relative to the housing 3 could also formed by only one or two larger rings (not shown).

On assembly of the container 4 at the housing 3, the cylindrical wall 28 of the proximal end region 9 of the housing 3 is inserted into the groove 33 of the container 4. The guidance of the container 4 relative to the housing 3 is further enhanced by aligning the pin 19 of the housing 3 with the longitudinal section 17' of the container 4.

The container 4 is then moved first in the direction of the longitudinal axis A in the direction of the wing-like projections 11 by guiding the cylindrical wall 28 of the proximal region 9 of the housing 3 in the groove 33 in a linear manner. Once the pins 19, 19' reach the end of the respective longitudinal section 17', the container is then rotated in the direction of the arrow B (see Fig. 1) such that the pin 19 is received in the connection section 18 and is stored there, i.e. the child safety lock of the discharger 1 is activated. This means that following the rotation of the discharger 1, the child safety lock is activated by means of the radial displacement and the discharger 1 arrives in its storage state.

On displacing the discharger 1 into the discharging state and subsequently arriving in the position shown in the part sectional drawing of Fig. 4, the discharged state, the piercing tip 24 exerts a substantially uniform pressure on the membrane 13 causing this to initially deflect and then be uniformly pierced starting from the center of the membrane 13 radially outwardly and then permitting the fluid F stored in the compartment 12 to arrive directly at the inlet 23 present in the piercing tip 24 in the region of the start of the piercing of the membrane 13. Due to the pressure increased in the compartment 12 on pressing the container 4 towards the wing-like projections 11, the fluid F is discharged via the inlet 23, the passage and the outlet 7 from the dispensing element 2.

As the container 4 is moved in the direction of the longitudinal axis A towards the wing-like projections 11, the container 4 is linearly guided relative to the housing 3. This guidance is brought about by means of the interaction taking place between the cylindrical wall 28, the guide groove 33 and the inner and outer walls 32, 14 of the container 4.

Moreover, following the piercing of the membrane 13 and during the further guidance of the cylindrical wall 28, relative to the guide groove 33 and the inner and outer walls 32, 14 of the container 4 towards the wing-like projections 11, the sealing lip 29 present at the inner surface 30 of the housing 3 is brought into engagement with an outer surface 36 of the inner wall 32 of the double walled container 4. By means of the engagement of the sealing lip 29 at the outer surface 36 a seal is formed between the proximal region 9 of the housing and the container 4. The seal thereby forms a barrier to the fluid F escaping from the housing 3 in the proximal region 9, namely via the guide groove 33 and the slot 16.

The sealing lip 29 is thereby configured to only engage the outer surface 36 of the inner wall 32 once the piercing tip 24 comes into contact with the membrane 13. The sealing lip 29 maintains contact with the outer surface 36 of the inner wall 32 even after the piercing tip 14 has moved past the original position of the membrane 13 towards the piercing tip receiving end 31.

In order to ensure that a sealing element 29' of the housing 3 engages the wall 32 of the container 4, a portion of the container 4, preferably a portion of the wall 32 of the container 4 is received within the housing 3.

As the container 4 is moved further in the direction of the longitudinal axis A, an inner surface 37 of the compartment 12 of the double walled container 4 is initially brought into contact with the first sealing lip 25. As the container 4 is moved further in the direction of the longitudinal axis A, the inner surface 37 comes into contact with the second sealing lip 26 and then with the third sealing lip 27. The sealing lips 25, 26, 27 thereby provide a seal between the static mixer 10 and the inner wall 32 of the compartment 12 of the container 4. This seal prevents a fluid F from arriving in the space 38 provided between the piercing tip 24 and the first of the three rings 35 and thereby from passing into a part of the housing 3 at the distal region 8 of the housing 3.

A side effect of the pressure increase of the fluid F prior to discharging is, in particular with regard to viscous fluids, such as frontline, that the fluid F can leak between the inlet 23 and the membrane 13 such that it runs along the piercing tip 24 and towards the distal region 8 of the housing 3. By providing the sealing elements 25', 26', 27' between the piercing tip 24 and the inner surface 37 of the compartment this movement of the fluid F into the distal region 8 of the housing 3 is avoided.

As can be seen from the part sectional drawing of Fig. 4, the first, second and third sealing lips 25, 26, 27 are in contact with the inner surface 37 of the compartment 12, thereby ensuring that no fluid F can pass from the compartment 12 via the piercing tip 24 and the first, second and third sealing lips 25, 26, 27.

Fig. 5 shows a section through a further kind of static piston 10 having the piercing tip 24. In this section one can see that the first sealing element 25' has a diameter that is smaller than a diameter of the second sealing element 26' and of the third sealing element 27'. The diameter of the second and third sealing elements 26', 27' is at least substantially identical.

In this connection it should be noted that an external diameter of the second and third sealing elements 26', 27' is larger than an internal diameter of the compartment 12. By way of example, the external diameter of the second and third sealing elements 26', 27' is selected to be 0.01 mm to 0.2 mm larger than the internal diameter of the compartment 12 of the container 4, preferably the external diameter of the second and third sealing elements 26', 27' is selected to be 0.05 to 0.15 mm larger than the internal diameter of the compartment 12 of the container 4.

It should further be noted that the external diameter of the first sealing element 25' is smaller than or equal to the internal diameter of the compartment 12. By way of example, the external diameter of the first sealing element 25' is selected to be 0.00 mm to 0.2 mm smaller than the internal diameter of the compartment 12 of the container 4, preferably the external diameter of the first sealing element 25' is selected to be 0.02 to 0.10 mm smaller than the internal diameter of the compartment 12 of the container 4.

In use of the static piston 10 the first sealing element 25' then pushes the pierced membrane 13 into the compartment 12 and a seal is initially effected between the membrane 13 and the first sealing element 25'. In this way the first sealing element clears the way for the second and third sealing elements 26', 27' in order to ensure an as good as possible seal between the inner surface 37 of the wall 32 of the compartment 12 and the piercing tip 24.

By forming the second and third sealing elements 26', 27' slightly larger than the internal diameter of the compartment 12, the second and third sealing elements 26', 27' are compressed on introduction into the compartment 12 in order to ensure an as good as possible seal between the inner surface 37 of the wall 32 of the compartment 12 and the piercing tip 24.

An axial distance (clearance) between the second and third sealing elements 26', 27' is selected to be in the range of 0.7 to 2 mm, in the present instance the axial distance amounts to 1 mm.

In the plane of the section shown in Fig. 5, a cross-section of the second and third sealing elements 26', 27' shows that these are formed as circumferentially extending sealing lips 26, 27 at a substantially triangular structure projecting from a central section 10' of the static piston 10. The central section 10' extends around the longitudinal axis A.

In contrast to this the first sealing element 25' is formed as a circumferentially extending sealing lip 25 at the end 24' of the piercing tip 24 remote from the end 24" comprising the inlet 23. The end 24' tapers from the sealing lip 25 towards the central section 10'

A Luer lock type connection 20 is formed at an end 23' of the static piston 10 remote from the inlet 23. This Luer lock type connection 20 can be used to connect various kinds (not shown) of dispensing elements 2 to the static piston 10 in a simple manner.

In this connection it should be noted that the connection formed between the dispensing element 2 and the static piston 10 can also be formed using a different form of connection other than a Luer taper, such as the Luer lock of Fig. 5, or the integral connection of Figs. 3 and 4 discussed in the foregoing. By way of example a bayonet type connection or a simple threaded connection etc. could also be provided as forms of connection between the dispensing element 2 and the static piston 10.

It should further be noted that the dispensing element 2 can be formed by a variety of components, for example, a spray head as shown in Figs. 3 and 4; a syringe type dispensing element, e.g. a needle; a dropper type dispensing element, e.g. a simple pipe having an aperture formed at its distal end (not shown), the aperture optionally being surrounded by a thickened portion to e.g. prevent stitching; and the like can be used as a dispensing element 2.

The dimensions, sizes and shapes of the first, second and third sealing elements 25', 26', 27' discussed in connection with Fig. 5 can naturally be employed at the static piston 10 shown and discussed in connection with Figs. 3 and 4.

It should be noted in this connection that the first, second and third sealing lips 25, 26, 27 are forms of sealing elements 25', 26', 27' and different kinds of sealing elements 25', 26', 27' other than the first, second and third sealing lips 25, 26, 27 could also be employed provided they provide the desired sealing function in this region of the discharger 1.

As can further be seen from Fig. 4, the fourth sealing lip 29 that is designed to engage the outer surface 36 of the inner wall 32 of the double walled container 4, to form a seal for the fluid F in this region, is compressed to such an extent that it is no longer visible. This level of compression is required, on the one hand, to ensure a seal in this region and, on the other hand, such that the cylindrical wall 28 can be reliably guided within the groove 33. The fourth sealing lip 29 is provided in order to ensure that if a fluid F were to pass the first, second and third sealing lips 25, 26, 27 that no fluid can escape from the discharger 1 between the housing 3 and the container 4.

The guide groove 33 is provided at the double walled container 4 in order to receive at least some of the housing 3, preferably some of the cylindrical wall 28 of the housing 3. In this way one can ensure that the container 4 is reliably guided relative to the housing 3 and more specifically with respect to the piercing tip 24 in order, to safeguard that the various sealing elements 25', 26', 27', 29', e.g. the sealing lips 25, 26, 27 and 29 etc., are engaged in a uniform manner in order to prevent liquids from leaking from the container 4 into a part of the housing 3 or to the outside in a non-desired manner.

In this connection it should be noted that the sealing elements 25', 26', 27', 29' can either be integrally formed and/or fixedly connected to the respective parts of the housing 3. Alternatively, they could be formed by separate sealing elements (not shown), such as an O-ring, that are then arranged at the respective position e.g. in a specifically provided groove (also not shown).

The improved guidance is brought about by forming a width of the guide groove 33 marginally wider than a thickness of the cylindrical wall 28 of the housing 3 in such a way that the cylindrical wall 28 is moveably received in the guide groove 33 with sufficient clearance to move, but yet not so much clearance that a play is present between the housing 3 and the container 4 allowing these components to become skew with respect to one another.

In this connection it should be noted that the clearance between the guide groove 33 and the cylindrical wall of the housing 3 is preferably selected to be 0.1 mm on both sides of the cylindrical wall 28 of the housing 3.

The clearance can however be selected to be 0 mm on one side of the cylindrical wall 28 of the housing 3 and 0.1 mm on the other side of the cylindrical wall 28. Such a varying clearance at either side of the cylindrical wall may be due to the selection of the material of the cylindrical wall 28 of the housing 3 or of one of the walls 32, 14 of the container 4 that interact with the cylindrical wall 28 of the housing 3.

A length of the guide groove 33 in the shown example corresponds to a length of cylindrical wall 28 from the wing-like projections 11 to the end 39 in the proximal region 9 of the housing 3. Moreover, a length of the guide groove 33 corresponds to approximately a length of the inner wall 32 of the container 4. The outer wall 14 of the container 4 is longer than the inner wall 32.

The length L of the compartment 12 formed within the inner wall 32 is shorter than the length of the inner wall 32. A depth of the piercing tip receiving end 31 of the compartment 12 corresponds to approximately 20 to 30% of the length L of the compartment 12.

As also shown in Fig. 4 the wing-like projections 11 of the housing 3 do not contact the outer wall 14 of the container 4. This is because the wing-like projections 11 are arranged at a height of the housing 3 relative to the longitudinal axis A such that the static piston 10 can come to a stop at the end of the compartment 12 of the container 4 and hence the point of interaction between the static piston 10 and the piercing tip receiving end 31 defines a stop for the container 4 and not the wing-like projections 11.

Generally speaking the discharger 1 and the container 4 described herein are manually operable dischargers and containers that are held in the hand of a user. They are typically designed as suitable for storing and/or administering a single dose of a fluid F, such as a drug, a medication or other kind of preparation used in the care of humans or animals. This single dose can be administered in one, two or several steps, e.g. if a fluid F is to be administered to the eyes or nostrils of a patient or animal.

### List of reference numerals:

- 1: discharger
- 2: dispensing element
- 3: housing
- 4: container
- 5: distal end
- 6: proximal end
- 7: outlet
- 8: distal region
- 9: proximal region
- 10: static piston
- 10': central section of 10
- 11: wing-like projections
- 12: compartment
- 13: membrane
- 13': plane comprising 13
- 13": seal
- 13'": front end
- 14: outer wall
- 14': end of 14
- 14": outer surface of 14
- 15: cut-out
- 16: slot
- 17, 17': longitudinal section
- 18: connection section
- 19, 19': pin
- 20: Luer lock
- 21: end
- 22: recess
- 23: inlet
- 23': end remote from inlet
- 24,: piercing tip
- 24', 24": end of 24
- 25: sealing lip
- 25': sealing element
- 26: sealing lip
- 26': sealing element
- 27: sealing lip
- 27': sealing element
- 28: cylindrical wall
- 28': outer surface of 28
- 29: sealing lip
- 29': sealing element
- 30: inner surface of 28
- 31: piercing tip receiving end
- 31': end of 12
- 31": point
- 32: inner wall
- 32': end of 32
- 33: groove
- 33': opening of 33
- 34: nose
- 35: ring
- 36: outer surface of 32
- 37: inner surface of 12
- 38: space
- 39: end
- 40: web
- 40': end of 33
- 41: wall
- 42: web
- 43: base

- A: longitudinal direction
- B: arrow
- F: fluid
- L: length

## Claims

1. A discharger (1) that comprises a dispensing element (2) and a housing (3), the housing (3) having a static piston (10) connected to the dispensing element (2) at least in a fluid conducting manner and with the static piston (10) having a piercing tip (24) and being arranged within the housing (3); the discharger (1) being configured to receive at least a portion of a container (4) for containing a fluid (F), with the container (4) then being moveable relative to the static piston (10) and the housing (3) along a longitudinal direction (A) of the discharger (1), wherein the housing (3) comprises a sealing element (29') arranged at an inner surface (30) of the housing (3), with the sealing element (29') projecting from the inner surface (30) of the housing (3) in the direction of the longitudinal direction (A) of the discharger (1).

2. A discharger in accordance with claim 1, wherein the housing (3) has a cylindrical wall (28) having the inner surface (30) and the sealing element (29') is arranged to circumferentially extend around the inner surface (30) of the cylindrical wall (28); and/or wherein the sealing element (29') is arranged at an inner surface (30) of the housing (3) in the region of the piercing tip (24).

3. A discharger in accordance with claim 1 or claim 2, wherein the sealing element (29') is an inwardly projecting sealing lip (29).

4. A discharger in accordance with at least one of the preceding claims,
wherein the sealing element (29') projects in the direction of the piercing tip (24) arranged along the longitudinal direction (A).

5. A discharger in accordance with at least one of the preceding claims,
wherein the sealing element (29') is arranged in a plane perpendicular to the longitudinal direction (A), with the plane further comprising at least one of the piercing tip (24), an end (24") of the piercing tip and an inlet (23) of the piercing tip (24).

6. A discharger in accordance with at least one of the preceding claims,
wherein the sealing element (29') is either integrally formed at the inner surface (30) of the cylindrical wall (28) or is in contact with the inner surface (30) of the cylindrical wall (28).

7. A discharger in accordance with at least one of the preceding claims,
wherein the static piston (10) comprises at least one further sealing element (25', 26', 27') arranged at the piercing tip (24).

8. A discharger in accordance with at least one of the preceding claims, further comprising the container (4), wherein the sealing element (29') is only in contact with or connected to the housing (3) in a storage state of the discharger (1); and
wherein the sealing element (29') is configured to engage the container (4) in at least one of a discharging state and a discharged state of the discharger (1) in addition to being in contact with or connected to the housing (3) in both the discharging state and the discharged state of the discharger (1).

9. A discharger in accordance with claim 8, wherein the container (4) comprises a seal (13"), with the piercing tip (24) being configured to pierce the seal (13") of the container (4) when the container (4) is moved out of the storage state and towards the housing (3), and in particular wherein the sealing element (29') is configured to engage the container (4), preferably a surface (36) of the container (4), as soon as the seal (13") of the container (4) is in contact with the piercing tip (24).

10. A discharger in accordance with at claim 9, wherein the static piston (10) comprises at least one further sealing element (25', 26', 27') arranged at the piercing tip (24) and the at least one further sealing element (25', 26', 27') provides a seal between the piercing tip (24) and an inner surface (37) of the container (4) when the moveable container (4) is moved relative to the housing (3) into a position in which the seal (13") is pierced, with said inner surface being (37) being different from the surface (36) of the container (4), in particular wherein the at least one further sealing element (25', 26', 27') is a sealing lip (25, 26, 27).

11. A discharger in accordance with claim 9 or claim 10, wherein the container (4) comprises a compartment (12) in its interior in which the fluid (F) is initially stored prior to piercing the seal (13"), and wherein said surface (36) is an outer surface (36) of a wall (32) of the compartment (12).

12. A discharger in accordance with claim 11, wherein the container (4) further comprises an outer wall (14) arranged in parallel to the wall (32) of the compartment (12) and a guide groove (33) present between the outer wall (14) and the wall (32) of the compartment (12), with said guide groove (33) being configured to receive at least some of the housing (3).

13. A discharger in accordance with at least one of the preceding claims,
wherein the discharger (1) comprises a child safety lock.

14. A discharger in accordance with at least one of the preceding claims 7 to 13, wherein a fluid (F) is stored in the container (4) in the storage state of the discharger (1) and wherein the seal (13") is in particular configured as a membrane (13), preferably wherein the discharger is filled with a fluid (F) selected from the group of members consisting of a topical medication, a medical fluid, a cosmetic and/or skin care preparation, a dental fluid, a veterinary fluid, an adhesive fluid, a disinfectant fluid, and combinations of the foregoing.

15. A method of discharging a fluid (F) from a discharger (1), in particular in accordance with at least one of the claims 1 to 14, the method comprising the steps of:
- guiding a container (4) in a longitudinal direction (A) of the discharger (1) towards a piercing tip (24) of a static piston (10) accommodated within a housing (3) of the discharger (1),
- piercing a seal (13") present at the container (4) by means of the piercing tip (24) causing the seal (13") to be pierced, in particular initially at a center of the seal (13"),
- bringing an outer surface (36) of the container (4) into engagement with at least one sealing element arranged at an inner surface (30) of the housing to prevent the fluid (F) from passing between the at least one sealing element (29') and the outer surface (36), and preferably
- guiding the container (4) further towards the static piston (10) to discharge the fluid (F) via an outlet (7) of the discharger (1).
